# EUROPÄISCHE PATENTSCHRIFT

(11) **EP 0 531 911 B1**
(45) Veröffentlichungstag und Bekanntmachung des Hinweises auf die Patenterteilung: **10.12.1997**
(21) Anmeldenummer: 92115226.0
(22) Anmeldetag: 05.09.1992
(51) Int. Cl.: C12N 5/00

(54) **Kultivierung von Vero-Zellen**
Culturing of Vero cells
Culture de cellules de Vero

(30) Priorität: 11.09.1991 EP 91115336
(43) Veröffentlichungstag der Anmeldung: 17.03.1993
(73) Patentinhaber: Doerr, Hans-Wilhelm, Prof. Dr. med., D-63303 Dreieich (DE)
(72) Erfinder: Cinatl, Jaroslaw, Dr., W-6000 Frankfurt/Main 70 (DE); Cinatl, Jindrich, Dr., W-6053 Obertshausen (DE)
(74) Vertreter: Laudien, Dieter, Dr.

(56) Entgegenhaltungen:
- FR-A- 2 400 063
- JOURNAL OF CELL SCIENCE, Band 86, 1986, London (GB); AS CURTIS et al., Seiten 9-24
- BIOLOGICALS, Band 19, Nr. 2, April 1991, Geneva (CH); J. CINATL jr. et al., Seiten 87-92
- CHEMICAL ABSTRACTS, Band 116, Nr. 25, 22 Juni 1992, Columbus, OH (US); J. CINATL jr. et al., Seite 392, AN 251546r

## Beschreibung

Die Erfindung betrifft die Kultivierung von Vero-Zellen, insbesondere die Kultivierung in serumfreiem Medium. Sie ist z.B. für die Kultivierung von adhärenten Vero-Zellinien einschließlich zur Züchtung von Viren auf infizierten Vero-Zellen geeignet.

Die Kultivierung von Säugetierzellen in einem chemisch definierten Medium ohne Proteininhalt ist seit mehr als 30 Jahren bekannt [1, 2], ebenso verschiedene Zellinien, die in proteinfreiem Medium etabiliert worden sind [3-13]. Medien, die als Handelsprodukte zur Verfügung stehen, um das Wachstum von Zellen in Abwesenheit von fremden Proteinen zu ermöglichen, sind erst kürzlich entwickelt worden [14, 15].

Diese bekannten proteinfreien Medien sind in erster Linie für die Züchtung von kontinuierlichen Zellinien in einer Suspension wie z.B. Hybridoma geeignet [10, 14, 15], andererseits häufig nicht geeignet, um das Zellwachstum in Monolayerkulturen zu fördern [23].

Dabei wurde dargestellt, daß die Eigenschaften der Kulturoberoberflächen das Wachstum der Monolayerkulturen in proteinfreiem Medium signifikant beeinflussen [6, 16, 17]. Rappaport et al. haben gefunden, daß die Adhäsion und das Wachstum von Zellen abhängig von einer kritischen Anzahl von negativen Ladungen auf den Oberflächen sind. Sie haben nach einem besonderen Verfahren Glasflaschen mit Alkali behandelt, dadurch wurde die Kultivierung verschiedener Zellinien ohne vorhergehende Anpassung in einem proteinfreien Medium ermöglicht [24]. Diese Technik zur Vorbereitung der Kulturoberfläche ist allerdings umständlich und auf das Material Glas beschränkt. Außerdem wurden unterschiedliche Kunststoffoberflächen von verschiedenen Lieferanten in Assays unter Verwendung von Zellen in serumhaltigen Medien und anderen Proteinzusätzen geprüft. Die Ergebnisse dieser Untersuchung zeigten, daß für das Wachstum der Zellen in protein-freiem Medium eine andere Qualität der Kulturoberfläche erforderlich ist als für das Wachstum der Zellen in serumhaltigem Medium.

Daß die Eigenschaften der Oberflächen aus Glas oder Kunststoff einen signifikanten Einfluß auf das Wachstum der Einzelschichtzellen in einem proteinfreiem Medium haben, ist also seit Jahren bekannt [6, 16, 17]. Der Literatur ist daher zu entnehmen, daß nicht nur die Zusammensetzung des Mediums sondern auch die Eigenschaften der Kulturoberflächen optimiert werden müssen, um ein ausreichendes Wachstum der Zellen in einem solchen Medium zu erreichen.

Polyvinylformal (PVF) ist erstmalig von Barski und Maurin als Kulturoberfläche für mehrere Zelltypen in Medien, die Serum, Embroyalextrakt und Tyrodes Salzlösung enthielten, benutzt worden. Andere Autoren verwendeten PVF vor einiger Zeit zur Züchtung von verschiedenen Zellinien in Medien mit Protein-Zusatz [26-28].

Im Handel erhältliche Wegwerfartikel aus Kunststoff einschließlich Polysterin (PS) besitzen meist eine negative Oberflächenladung. Maroudas hat nachgewiesen, daß für die Ausbreitung der Zellen eine Dichte der negativen Ladung von 2-10x10¹⁴ per cm² am günstigsten ist [18, 19], z.B. hat PS-TC eine negative Ladung von 4-7x10¹⁴ per cm². Andererseits hat PVF eine negative Ladung von nur 0.4-0.8x10¹⁴ per cm². Aufgabe der Erfindung ist es, eine neue Kulturoberfläche zur Verfügung zu stellen, in der Vero-Zellen, vorzugsweise als kontinuierliche Monolayer, in einem proteinfreien Medium kultiviert werden können.

Diese Aufgabe wird überraschenderweise durch die Verwendung von PVF und/oder PVB als Zellsubstrat erfüllt.

Gegenstand der Erfindung ist daher ein Verfahren zur Kultivierung von Vero-Zellen in proteinfreiem Medium, dadurch gekennzeichnet, daß man Kulturoberflächen aus Polyvinylformal und/oder Polyvinylbutyral verwendet.

Polyvinylformal (PVF) und Polyvinylbutyral (PVB) sind bekannte Polymere der Gruppe "Polyvinylacetals", die durch die Reaktion von Aldehyden (Formaldehyd oder Butyraldehyd) und Polyvinylalkohol entstehen können. Bekanntlich kann man durch Anwendung von entsprechenden Mengen der Ausgangsmonomere PVF und PVB mit verschiedenen gezielten Inhalten von z.B. Hydroxylgruppen erhalten, z.B. enthalten die Handelsprodukte Butvar®B-98 (Monsanto Company, Texas) 20% Hydroxyl als Polyvinylalkohol und Butvar®B-76 13% Hydroxyl unter den gleichen Voraussetzungen; verschiedene Formvar®-Harnstoffe wie z.B. Formvar®5/95E und 15/95E enthalten nur 6.5% Hydroxy als Polyvinylalkohol. Butvar® ist damit hydrophiler als Formvar®. PVF und PVB können allein oder als Mischungen verwendet werden.

Vorzugsweise werden die Vero-Zellen als kontinuierliche Monolayerkulturen kultiviert.

In einem bevorzugten Verfahren gemäß der Erfindung werden die Vero-Zellen im proteinfreien Medium ohne Adaptionsphase gezüchtet.

Als proteinfreies Medium kann man z.B. ein 1:1 modifiziertes DMEM/F12-Medium (ergänzt mit Spurenelementen, C-Vitamin Phosphat und Glutamindipeptid), Ham'sF10, Ham'sF12, α-MEM oder DMEM/F12 ohne Spurenelemente und C-Vitamine verwenden. Bevorzugt wird die oben erwähnte modifizierte Mischung aus DMEM F12.

Eine weitere bevorzugte Medienzusammensetzung, die z.B. die Wachstumskinetik der Vero-Zellen verbessern lassen kann, ist eine Mischung der zwei folgenden Medien:
1. Hybridmax (Sigma) und
2. SRE-199 (Sigma)
Mischung 1:1 plus 800 g/ml CaCl₂.

Für den Fachmann ist es einfach, die verschiedenen zur Verfügung stehenden Medien in diesen Verfahren analog der Beispiele zu testen, um die passenden Medien zu identifizieren.

Vorzugsweise werden die Zellen in einer ursprünglichen Zellendichte von mindestens 2.0x10⁴ Zellen/cm² inkubiert.

Weiterer Gegenstand der Erfindung zur Kultivierung der Vero-Zellen in protein-freiem Medium ist die Verwendung eines Kultivierungsreaktors, der eine Kulturoberfläche aus PVF und/oder PVB enthält.

Als Kulturoberfläche, beschichtet mit PVF und/oder PVB, können verschiedene Füllungskörper wie z.B. Sattel, Stahl-Spiralen, Blas- bzw. Keramikbeads usw. dienen.

Obwohl das Kulturgefäß oder der Füllkörper ganz aus PVF bestehen können, wird normalerweise eine dünne Schicht aus PVF auf eine Grundstruktur aus Metall, Glas oder Kunststoff, z.B. nach bekannten Methoden, abgelagert. Vorzugsweise wird die beschichtete Oberfläche durch ein Verfahren hergestellt, mit dem man die Oberfläche einer Grundstruktur in Kontakt mit einer Lösung von PVF bringt und das Lösungsmittel verdampft oder verdampfen läßt.

Das Verfahren der Erfindung eignet sich auch zur Herstellung von biologischen Substanzen durch Kultivierung von Vero-Zellen, die die Substanz als endogenes Protein oder als rekombinantes Protein produzieren. Zu diesem Zweck kann z.B. eine durch ein für ein fremdes Protein kodierendes Gen transformierte Vero-Zelle auf der PVF und/oder PVB-Kulturoberfläche im proteinfreien Medium kultiviert werden und nach Ende der Kultivierung das Protein durch an sich bekannte Methoden isoliert und gereinigt werden. Als biologische Substanzen kommen z.B. t-PA, EPO, hGH, ICAM-1, humane Lungensurfactant-Proteine wie z.B. SP-B, SP-C usw. in Frage.

Das Verfahren eignet sich auch zur Herstellung von Viren bzw. Vakzinen, wobei eine mit einem Virus (z.B. Poliovirus, Adenovirus, BVD) infizierte Vero-Zelle auf der PVF und/oder PVB-Kulturoberfläche in proteinfreiem Medium kultiviert wird und die Viren nach Beendigung der Kultivierung durch an sich bekannte Methoden isoliert und nachbehandelt wird.

Fig. 1 zeigt die stilisierten Formeln von PVF mit Hydroxyl Gruppen, Acetat-Gruppen und Formal-Gruppen, die willkürlich entlang der Moleküle verteilt sind. Die Formar-Verbindung von PVF enthält 5-5.6% Polyvinylalkohol, 9.5-13% Polyvinylacetat und 82% Polyvinylformal.

Fig. 2 zeigt die Wachstumskurven von Vero-Zellen in MEM, mit 10% FBS (-x-) ergänzt, und von Vero-Zellen in dem protein-freien Medium auf PS-TC- (-Δ-), auf Poly-D-Lysine- (-*-) und auf PVF-Oberflächen (-□-).

Fig. 3 zeigt das morphologische Erscheinungsbild von Vero Zellen im proteinfreien Medium auf PS-TC-Oberflächen (A), Poly-D-Lysine-Oberflächen (B) und PVF-Oberflächen (C). 9 Tage nach der Beimpfungphase unter dem Kontrast-Mikroskop.

Fig. 4 zeigt die Langzeitkultivierung von Vero-Zellen in proteinfreiem Medium an PVF-beschichteten Oberflächen. Die Zellen wurden in Intervallen von 9 Tagen unter Verwendung einer Kollagenase-Dispase-Lösung subkultiviert und in einer Dichte von 2.5x10⁴ Zellen per cm² eingesät.

Fig. 5 zeigt das morphologische Erscheinungsbild von Vero-Zellen nach 40 Passagen im proteinfreien Medium an PVF-beschichteten Oberflächen. Die Zellen wurden mit May-Grünwald-Giemsa gefärbt.

### Beispiel 1

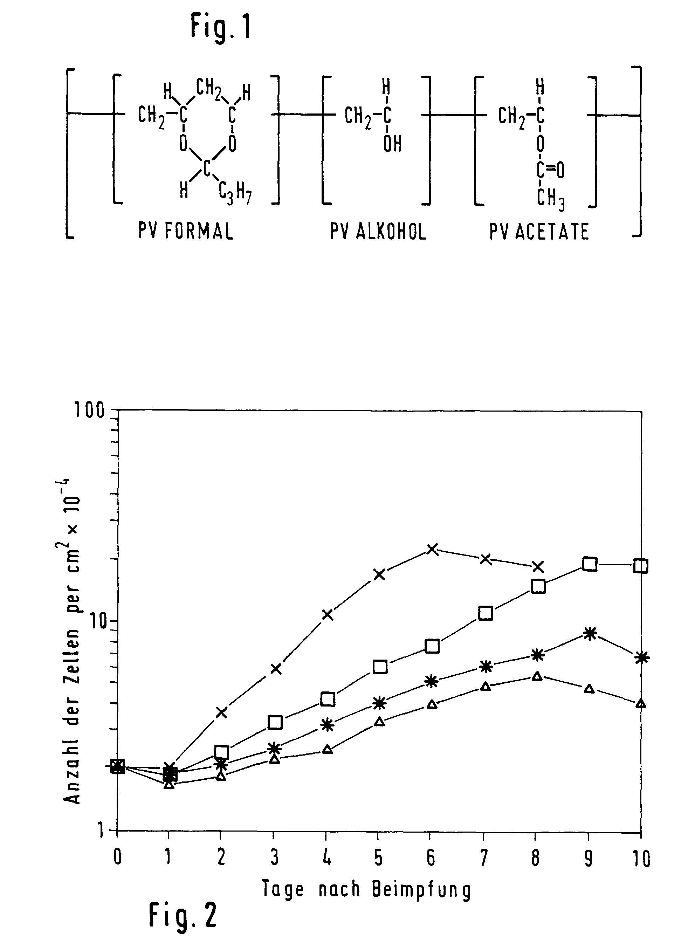

Eine Polyvinylformal-Lösung erhält man durch die Auflösung von pulverisiertem Polyvinylformal (PVF, Fig. 1) (Formvar^{(R)}; Monsanto Company, Texas, USA) in Essigsäure in verschiedenen Konzentrationen von 1 bis 20 mg/ml. Die resultierende Lösung wurde in Mengen von 5 ml oder 2 ml auf die Kulturoberfläche von jeweils 25 cm² PS-TC-Kolben (Falcon, Heidelberg, BRD, und Greiner, Nürtingen, BRD) und Schalen (Greiner, Nürtingen, BRD) gegeben; nach 2 Minuten wurde die Lösung abgegossen. Die Kolben wurden 24 Stunden lang in einen Laminar Flowhood gestellt, um den Rest der Essigsäure verdampfen zu lassen. Danach wurden die Kolben mit doppeltdestilliertem Wasser gewaschen und entweder sofort zur Zellkultur verwendet oder bis zu 3 Monate nach ihrer Vorbereitung.

Die negative Ladungsdichtigkeit der Oberflächen der Gefäße wurde mittels einer kationischen Farbstoffbindung bestimmt (wie bei Maroudas beschrieben [10, 19]).

Es wurde angenommen, daß die Anzahl der Moleküle, die an die Oberfläche gebunden sind, mit der absoluten Anzahl der festen negativen Ladungen identisch sind.

Als Zellinien wurden murine NCTC Klon 929L und Swiss 3TC, syrische Hamster BHK-21 Klon 13, chinesische Hamster CHO-Kl, Schweine PK-15, Hunde MDCK, Affen Vero und LLC MK2, humanes A431 und HeLa-Zellinien von der American Type Culture Collection verwendet.

Stockkulturen dieser Zellen, mit Ausnahme der CHO-Zellen, wurden in MEM enthaltendem 5-10% Fetal Bovine Serum (FBS) vermehrt. Die CHO-Zellen wurden in Ham's F12, 10% FBS enthaltend, gezüchtet.

Alle Zellen wurden in Kulturflaschen mit einer Kulturoberfläche von 75 cm² in einem CO₂ Inkubator (37°C, 5% CO₂) kultiviert. Die Zellen wurden routinemäßig kontrolliert, um Mycoplasma mittels der Hoechst 33258 DNA Staining-Methode zu identifizieren. Alle Färbungen waren negativ.

Das proteinfreie Medium war eine modifizierte 1:1-Mischung aus DMEM-F12, enthaltend 15 mM HEPES und 1.2 g/l Natrium bicarbonat (Tabelle 1). Die Verwendung von Glycyl-L Glutamine anstelle von L-Glutamine hatte eine erhöhte Konzentration von Glycin in dem Medium zur Folge. Diese erhöhte Konzentration von Glycin hatte aber keinen Einfluß auf die Vermehrung der verschiedenen Zellen.

Um die proteinfreien Zellkulturen in Gang zu setzen, wurden die konfluenten Kulturen dreimal mit PBS gewaschen und von der Kulturoberfläche unter Verwendung einer 2:1 Mischung aus Kollagenase H 0.25% und Dispase 0.25% in PSB freigesetzt. Die freigesetzten Zellen wurden dreimal mit PBS gewaschen und in proteinfreiem Medium innokuliert und anschließend im CO₂-Inkubator (37°C, 5% CO₂) inkubiert.

Um das Zellwachstum und die Adhäsion zu messen, wurden die Zellen dreimal mit PBS gewaschen und zwar in unterschiedlichen Zeitabständen nach der Innokulation, von der Kulturoberfläche mittels Trypsin 0.1% in PBS freigesetzt und die vitalen Zellen mittels eines Hemocytometers ausgezählt.

Die Lebensfähigkeit der Zellen wurde mittels der FarbstoffExclusion-Methode ermittelt, als Farbstoff wurde 0.5% Trypan-Blau-Lösung verwendet.

Die Sättigungsdichte der Zellpopulation stellt die maximale Anzahl der Zellen dar, die unter spezifischen Kulturbedingungen [22] erreicht werden kann. Der Wert der Populations-Verdoppelung (PDL) wurde aus der ursprünglichen Zahl, die als Impfung benutzt wurde (Ni), und der Zahl der geernteten Zellen (Nf) unter Benutzung der Formel PDL = 3.32 log (Nf/Ni) [22] ermittelt.

### Beispiel 2

Polioviren der Typen 1 bis 3, klinische Isolate, und Adenoviren der Typen 2 und 5 wurden aus adenoidem Gewebe isoliert. Die Identität der Viren wurde mittels der Serum-Neutralisations-Untersuchung unter Verwendung der standardisierten Polioviren der Typen 1 bis 3- und Adenoviren der Typen 2 und 5-Antisera festgestellt. Alle Viren wurden in Verozellen propagiert und titriert, die in MEM, 2% FBS enthaltend, gehalten wurden.

Um eine Kontaminierung des proteinfreien Mediums durch Serumproteinen aus dem obigen Virenstamm zu vermeiden, wurden nur intrazellulare Viren der infizierten Kulturen in der folgenden Untersuchung verwendet.

Die infizierten Zellen wurden von der Kulturoberfläche gekratzt, dreimal mit PBS gewaschen und jedesmal in neuem proteinfreiem Medium suspendiert. Die Zellen wurden danach mittels Utraschall lysiert, zentrifugiert (1200 xg) und die Viren enthaltenden Überstände wurden bei -70°C gefroren.

Konfluente Kulturen der Vero-Zellen, die in 35 mm Kulturschalen mit PVF-Oberflächen in proteinfreiem Medium kultiviert wurden, wurden 5 mal mit PBS gewaschen und wurden dann mit 0.2 ml der Virensuspension, die Polioviren mit MOI 0.01 PFU/Zellen und Adenoviren mit 0.05 PFV/Zellen enthalten, geimpft. Nach einer Stunde bei 37°C wurde das Inokulum entfernt, die Zellen mit PBS gewaschen und 2 ml proteinfreies DMEM/F12 zugesetzt. Nach 3 Tagen wurden die Überstände gesammelt und Virustitration durch Plaqueassay vorgenommen. Zu diesem Zweck wurden die Überstände der infizierten Kulturen verdünnt (in zweifachen Schritten).

Dazu wurden konfluente Verozellkulturen mit PBS gewaschen und die mit 0.2 ml der verdünnten Virussuspension geimpft. Nach 1 Stunde Inkubation wurde das Inokulum entfernt und die inokulierten Zellen mit PBS gewaschen. Die so infizierten Verozellen wurden zur Titerbestimmung 4 Tage (Polio) oder 7 Tage (Adeno) mit 2 ml 2%iger Carboxymethylzellulose in MEM + 2% FCS bei 37°C (CO₂-Inkubator) gehalten. Pro Verdünnung wurden 3 Kulturschalen angesetzt. Nach der entsprechenden Inkubationszeit wurden die Zellen gewaschen (PBS) und mit 0.2% Kristallviolett gefärbt und die Titer bestimmt.

### Ergebnis:

In Tabelle 2 wird die Wirkung der PVF-Oberfläche auf das Wachstum von zehn kontinuierlichen Zellinien in protein-freiem DMEM-F12 mit der Wirkung von PS-TC oder Poly-D-Lysine verglichen.

Alle Zellen wurden bei einer Dichte von 2x10⁴ Zellen/cm² eingesät. Die Ergebnisse entsprachen der Sättigungsdichte der Zellen in den verschiedenen Zeitabständen zwischen 5 und 9 Tagen.

3T6 war die einzige Zellinie, die minimal auf der PVF anhaftete und keine wesentliche Ausbreitung zeigte, während 3T6-Zellen auf PS-TC oder Poly-D-Lysine direkt nach ihrer Adhäsion eine Anfangsproliferation aufweisen.

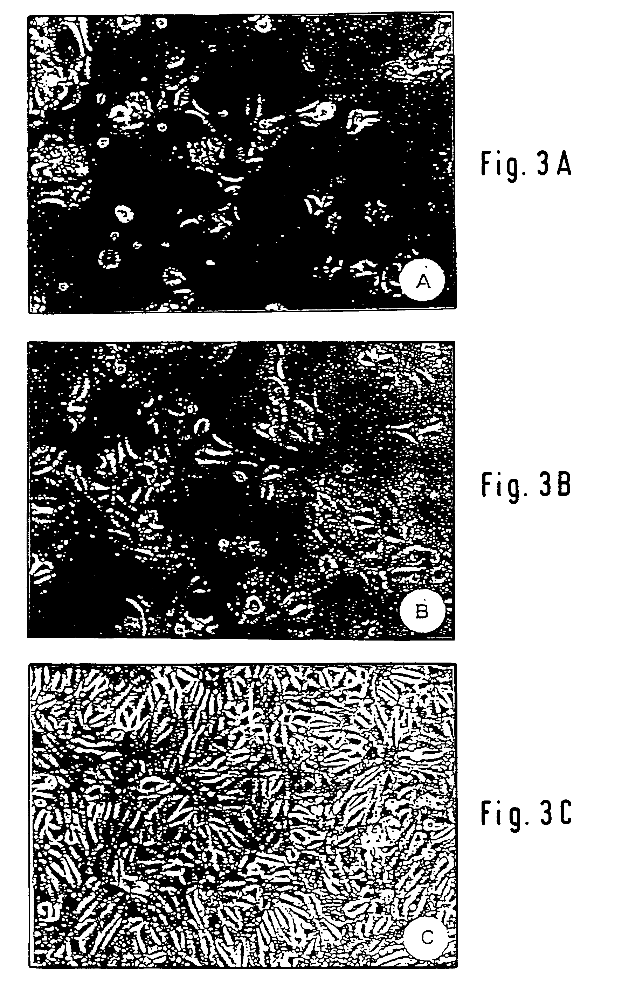

Sechs Zellinien einschließlich der NCTC Klon 929L, BHK, CHO-Kl, PK 15, A431 und HeLa weisen die gleiche Ausbreitung auf PVF und auf den handelsüblichen PS-Flaschen auf. In den meisten Fällen konnte man das Zellwachstum durch die Behandlung einer Oberfläche von PS-TC-mit Poly-D-Lysine steigern, eine Konfluenz von mehr als 75% kann damit nicht erreicht werden. Zwei Zellinien einschließlich MDCK und LLC-MK2 konnten sich wesentlich besser auf PVF als auf PS-TC ausbreiten. Die Ausbreitung von MDCK auf PVF wurde im Vergleich zu den Zellen auf der aus Poly-D-Lysine bestehenden Oberfläche etwas gesteigert. Diese Zellen konnten jedoch auch weder eine konfluente Zellschicht noch ein kontinuierliches Wachstum erreichen. Demgegenüber wuchsen die Vero-Zellen viel besser auf PVF als auf aus PS-TC oder Poly-D-Lysine beschichteten Oberflächen. Am 9. Tag nach der Einsaat waren die Zellen konfluent gewachsen (Fig. 2 und 3). Die Verdoppelungszeit der Vero-Zellen auf PVF im proteinfreien Medium wurde nach 48 Stunden erreicht, in DMEM-F12, 10% FBS enthaltendem Medium nach 28 Stunden. Die Sättigungsdichte der Zellen unter Verwendung von serum-haltigem Medium war nur ungefähr 17% höher als jene unter Verwendung von protein-freiem Medium auf PVF (Fig. 2).

In Tabelle 3 ist die Wirkung der verschiedenen Oberflächen auf die Adhäsion von Vero-Zellen in dem proteinfreien Medium dargestellt. Die Zellen an den Oberflächen von Poly-D-Lysine und PVF hefteten sich an und verbreiteten sich schneller als an den PS-TC enthaltenden Oberflächen. Dies konnte 30 Minuten nach der Beimpfung klar gesehen werden. Die Zahl der an den PS-B-Oberflächen gebundenen Zellen entsprach der Zahl der Zellen an PS-TC; darüber hinaus wurde eine beginnende Ausbreitung beobachtet.

Die in Tabelle 4 dargestellten Ergebnisse zeigen auf, daß die Ausbreitung der Vero-Zellen in dem protein-freien Medium stark abhängig von der zahl der eingesäten Zellen ist, wobei nur im Fall einer ursprünglichen Vero-Zellen-Dichte von über 0.2x10⁴ Zellen/cm² sich die Zahl der Zellen steigern konnte. Eine geringe beginnende Ausbreitung von Vero-Zellen zeigte sich an PS und Poly-D-Lysine, aber nur bei einer Einsaat mit einer anfänglichen Dichte von jeweils 2x10⁴ Zellen/cm² und 1x10⁴ Zellen/cm².

Tabelle 5 stellt das Wachstum der Vero-Zellen in protein-freiem Medium DMEM-F12 an PVF-beschichteten Oberflächen in Abhängigkeit verschiedener Formvar-Konzentrationen dar. Die Sättigungsdichten auf PVF-Oberflächen auf der Basis von Formvar-Lösungen mit Konzentrationen in den Bereichen von 0.5% bis 2% unterscheiden sich nicht wesentlich. Demgegenüber wurde eine signifikante Abnahme der Sättigungsdichte der Zellen im Fall von PVF mittels 0.1%iger Formvar-Lösung beobachtet.

Aus Tabelle 6 kann man entnehmen, daß PVF-enthaltende Oberflächen mindestens bis zu 3 Monate nach ihrer Vorbereitung ohne Abnahme der Wachstumsrate der Vero-Zellen in dem proteinfreien Medium verwendet werden können.

Die Messung der negativen Oberflächen-Ladungsdichte weist signifikante Unterschiede zwischen PS-TC- und PVF-behandelten Oberflächen auf. Andererseits hat man keine wesentlichen Unterschiede zwischen PVF und PS-B beobachtet. Der Bereich der Oberflächen-Ladungsdichte betrug 4-7x10¹⁴ per cm² für PS-TC, 0.3-0.7x10¹⁴ per cm² für PS-B und 0.4-0.8x10¹⁴ per cm² für PVF.

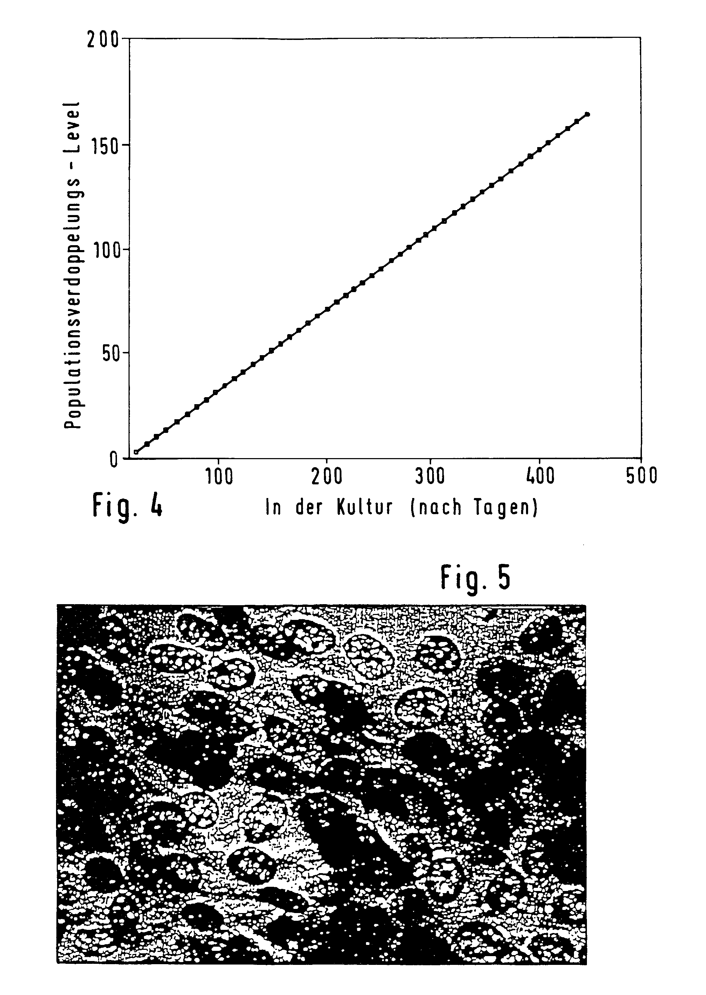

Die Sub-Kultivierung von Vero-Zellen in proteinfreiem Medium auf PVF konnte durchgeführt werden, ohne eine Abnahme der Wachstumsrate in aufeinanderfolgenden Durchführungen festzustellen, wobei bis zu fünfzig Sub-Kultivierungen, die zusammen 164 Populationsverdoppelungen erreichten, durchgeführt wurden. Das morphologische Erscheinungsbild der in dem proteinfreien Medium etablierten Vero-Zellen unterscheidet sich nicht von jenem der Zellen in entsprechendem serumhaltigen Medium (Fig. 5). Darüber hinaus stellt Fig. 5 dar, daß keine Hintergrundfärbung nach der Färbung mit May-Grünwald-Giemsa auftritt.

Die PVF-Schicht in obigem Beispiel besitzt optische Eigenschaften, die jener der PS-Kulturkolben ähnlich sind. Darüber hinaus ist nach Färbung mit May-Grünwald-Giemsa keine Hintergrundfärbung von PVF zu beobachten. Wir haben mehrere PVF-Zubereitungen getestet, die im Anteil an Hydroxy und Acetat unterschiedlich waren, jedoch ähnliche Wirkungen in der Ausbreitung der Vero-Zellen aufzeigten. Durch die Eigenschaften und die geringen Kosten von PVF eignet es sich als Kulturoberfläche für Vero-Zellen. Da Vero-Zellen oft in der Produktion von Virusimpfstoff oder anderen biologisch aktiven Substanzen Verwendung findet, können z.B. PVF-beschichtete Microcarrier der Herstellung von biologischen Substanzen dienen.

In dieser Untersuchung verwendeten wir relativ einfache proteinfreie Medien für die verschiedenen Zellinien. Denkbar ist, daß eine Verbesserung der protein-freien Medien ein Wachstum der Vero-Zellen an PVF noch steigern würde. Es muß berücksichtigt werden, daß ein erfolgreiches Wachstum von Zellen in proteinfreien Medien von verschiedenen Faktoren einschließlich Reinheit des Wassers, chemisch reine Glas- und Kunststoffware und beste Qualität der Chemikalien abhängt [6, 16, 17, 29]. Wir ergänzten das proteinfreie Medium mit Glycin-L-Glutamin anstatt mit L-Glutamin [30] und mit L-Ascorbinsäure-2-Phosphat [31]. Es ist zu vermuten, daß die Verwendung dieser stabilen Substanzen die Verbreitung von Vero-Zellen in proteinfreien Medien erhöht.

Wie oben in dem Beispiel bereits hingewiesen, wachsen nicht alle Zelltypen auf PVF so hervorragend wie in die Vero-Zellen.

**TABELLE 1**

| Substanzen mit proteinfreiem DMEM-F12 | | |
|---|---|---|
| Substanz | Konzentration im Medium mg/l | Quelle* |
| CoCL₂.6H₂O | 0.0002 | S |
| CuSO₄.5H₂O | 0.00025 | S |
| MnSO₄.7H₂O | 0.000024 | SE |
| Na₂SeO₃ | 0.0005 | S |
| Na₂SiO₃.9H₂O | 0.0065 | I |
| NH₄VO₃ | 0.00006 | SE |
| NiCl₂.6H₂O | 0.000012 | SE |
| (NH₄)₆Mo₇O₂₄.H₂O | 0.00012 | SE |
| SnCl₂.2H₂O | 0.00001 | S |
| L-Ascorbinnsäure 2-Phosphat-Magnesiumsalz | 28.95 | W |
| Glycyl-L-Glutamin | 800.00 | PK |

| | | |
|---|---|---|
| * S = Sigma; SE = Serva (Heidelberg, BRD); I = ICN Biochemicals (Mechenheim, BRD); W = Wako Pure Chemical Industrie, Ltd. (Osaka, Japan); PK = Pfrimmer - Kabi (Erlangen, BRD) | | |

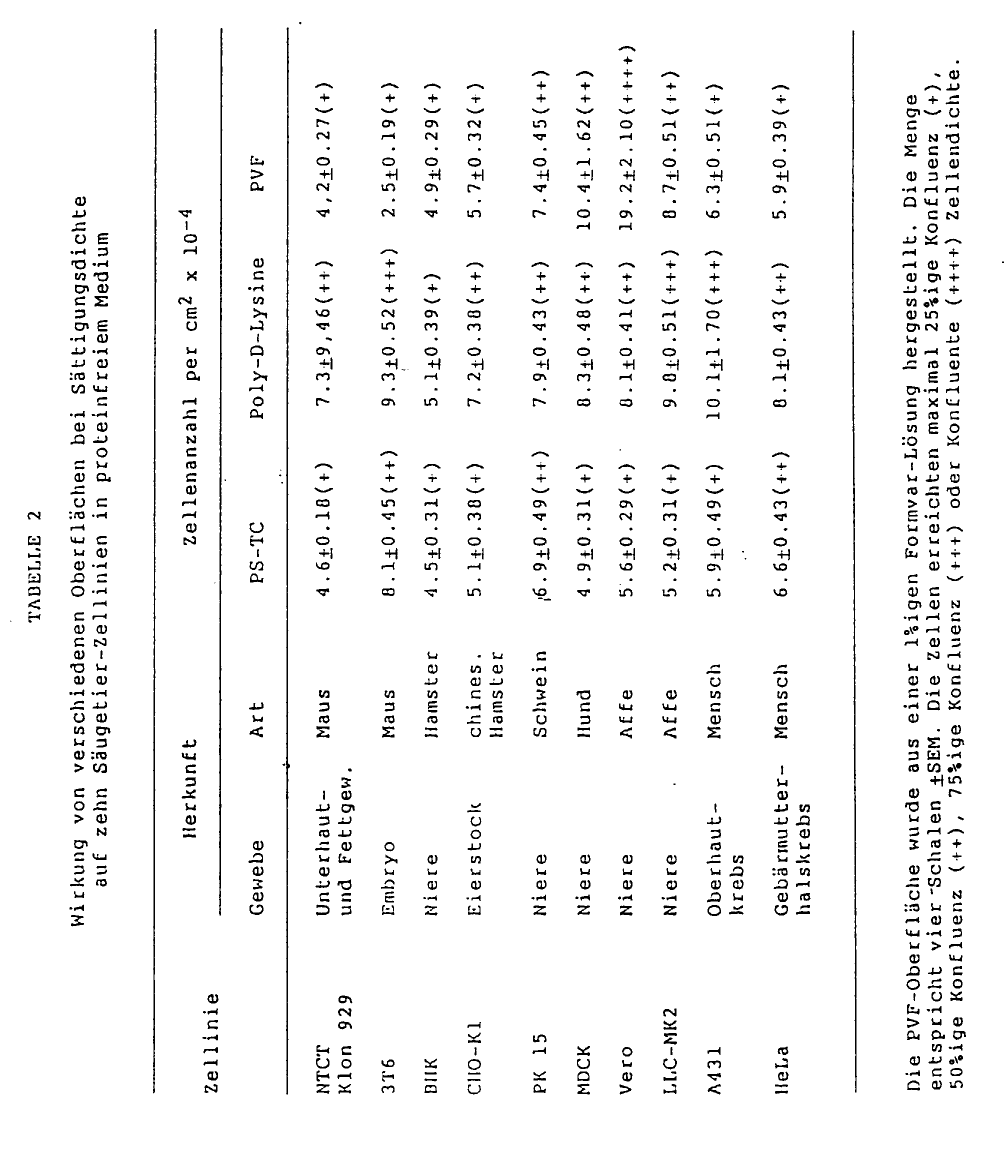

**TABELLE 3**

| Wirkung der verschiedenen Oberflächen auf die Adhäsion der Vero-Zellen im proteinfreien Medium | | | | |
|---|---|---|---|---|
| Zeit nach Beimpfung (Stunden) | Zellenanzahl/cm² x 10⁻⁴ | | | |
| | PS-B | PS-TC | Poly-D-Lysine | PVF |
| 30 | 1.31±0.21 | 1.49±0.23 | 1.81±0.25 | 1.74±0.19 |
| 60 | 1.56±0.19 | 1.61±0.21 | 1.78±0.21 | 1.86±0.23 |
| 90 | 1.61±0.22 | 1.68±0.26 | 1.83±0.18 | 1.85±0.25 |

Die PVF-Oberfläche wurde aus einer 1%igen Formvar-Lösung hergestellt. Die Menge entspricht vier Schalen ±SEM.

**TABELLE 4**

| Wirkung der Ausgangszelldichte auf die Ausbreitung der Vero-Zellen an der PVF-Oberfläche in proteinfreiem Medium | | | |
|---|---|---|---|
| Ausgangs- Zelldichte per cm² x 10⁻⁴ | Zelldichte per cm² x 10⁻⁴ nach Abschluß | | |
| | PS-TC | Poly-D-Lysine | PVF |
| 2 | 5.5±0.29 | 8.9±0.45 | 18.9±2.9 |
| 1 | 2.2±0.21 | 4.6±0.31 | 14.2±2.1 |
| 0.5 | 0 | 0 | 6.9±0.4 |
| 0.2 | 0 | 0 | 3.8±0.3 |
| 0.1 | 0 | 0 | 0 |

Die PVF-Oberfläche wurde aus einer 1%igen Formvar-Lösung hergestellt. Die Menge entspricht vier Schalen ±SEM.

**TABELLE 5**

| Wirkung der PVF-Oberfläche von Formvar-Lösungen verschiedener Konzentrationen auf die Sättigungsdichte von Vero-Zellen im proteinfreiem Medium | |
|---|---|
| PFV-Konzentration in % | Zellenanzahl per cm² x 10⁻⁴ |
| 0.10 | 12.5±2.5 |
| 0.50 | 18.6±3.1 |
| 1.00 | 18.3±2.8 |
| 2.00 | 18.9±2.4 |

Die Menge entspricht vier Schalen ±SEM. Die Zellen wurden 9 Tage nach Beimpfung ausgezählt.

**TABELLE 6**

| Wirksamkeit der PVF-Oberflächen bei Lagerung in verschiedenen Zeitintervallen bei Sättigungsdichte der Vero-Zellen in proteinfreiem Medium | |
|---|---|
| Tage nach Herstellung | Anzahl der Zellen/ cm² x 10⁻⁴ |
| 1 | 19.1±2.3 |
| 30 | 18.1±2.1 |
| 90 | 18.7±2.9 |

Die PVF-Oberflächen wurden von einer 1%igen Lösung Formvar hergestellt. Die Werte entsprechen denen von vier Schalen ± SEM. Die Zellen wurden 9 Tage nach Aussaat gezählt.

### References:

1. Evans, V.J., Bryant, C.J., Fiaramonti, M.C, McQuilkin, W.T., Sanford, K.K., and Earle, W.R. (1956) Cancer Res. 16, 77-86
2. Evans, V.J., Kerr, H.A., McQuilkin, W.T., Earle, W.R., and Hull, R.N. (1959) Am. J. Hyg. 70, 297-302
3. Evans, V.J., LaRock, J.F., Yosida; T.H., and Pottre, M. (1963) Exp. Cell Res. 32, 212-217
4. Cinatl, J., and Vesely, P. (1967) Folia Biologica 13, 61-67
5. Takoaka, T., and Katsuta, H. (1971) Exp. Cell Res. 67, 295-304
6. Sanford, K.K., and Evans, V.J. (1982) J. Natl. Cancer Ins. 68, 895-913
7. Okabe, T., Fujisawa, M., and Takaku, F. (1984) Proc. Natl. Acad. Sci. USA 81, 453-455
8. Yamaguchi, N. Morioka, H., Ohkura, H., Hirohashi, S., and Kawai, K. (1985) J. Natl. Cancer Inst. 75, 29-35
9. Yamaguchi, N., and Kawai, K. (1986) Cancer Res. 46, 5353-5359
10. Kovar, J., and Franek, F. (1987) Biotechnol. Lett. 9 ), 259-264
11. Cinatl, J. jr., cinatl, J., Gerein, V., Kornhuber, B., and Doerr, H.W. (1988) J. Biol. Stand. 16, 249-257
12. Hill, M., Hillova, J., Mariage-Samson, R., Fasciotto, B., and Krsmanovic, V. (1989) In Vitro Cell. Dev. Biol. 25, 49-56
13. Rikimaru, K., Toda, H., Tachikawa, N., Kamata, N., and Enomoto, S. (1990) In Vitro Cell. Dev. Biol. 26, 849-856
14. Fike, R., Pfohl, J., Jayme, D., and Weiss, S. (1990) In Vitro Cell. Dev. Biol. 26, 54A
15. Darfler, F.J. (1990) In Vitro Cell. Dev. Biol. 26, 769-778
16. Price, F.M., and Sanford, K.K. (1976) Procedure 10311. TCA Manual 2, 379-382
17. Cinatl, J. jr., Türk, M., Cinatl, J., Ahn, J.W., Rabenau H., and Doerr, H.W. (1990) In Vitro Cell. Dev. Biol. 26, 841-842
18. Maroudas, N.G. (1975) J. Theor. Biol. 49, 417-424
19. Maroudas, N.G. (1976) J. Cell. Physiol. 90, 511-520
20. Chen, T.R. (1977) Exp. Cell Res. 104, 255-262
21. McKeehan, W.L., McKeehan, K.A., Hammond, S.L., and Ham, R.G. (1977) In Vitro Cell. Dev. Biol. 13, 399-416
22. Schaeffer., W.I. (1984) In Vitro Cell. Dev. Biol. 20, 19-24
23. Kovar, J. (1989) In Vitro Cell Dev. Biol. 25, 395-396
24. Rappaport, C., Poole, J.P., and Rappaport, H.P. (1960) Exp. Cell Res 20, 465-510
25. Barski, G., and Maurin, J. (1948) Annal. Inst. Pasteur 74, 312-322
26. Furusawa, K., Suda, Y., and Tsuda, K. (1980) Kenkyu Hokoku-Sen'i Kobunshi Zairyo Kenkyosho 124, 17-21
27. Craig, A.M., Smith, J.H., and Denhardt, D.T (1989) J. Biol. Chem. 264, 9682-9689
28. Nordby, A., Tredt, K.E., Halgunset, J., Kopstad, G., and Haugen, O.A., (1989) Invest. Radiol. 24, 703-710
29. Cinatl, J. jr., Cinatl, J., Rabenau, H., and Doerr, H.W. (1989) J. Tissue Cult. Methods 12, 67-72
30. Roth, E., Ollenschlager, G., Hamilton, G., Simmel, A., Langer, K., Fekl, W., and Jakesz, R. (1988) In Vitro Cell. Dev. Biol. 24, 696-698
31. Hata, R., and Senoo, H. (1989) J. Cell. Physiol. 138, 8-16

## Patentansprüche

1. Verfahren zur Kultivierung von Vero-Zellen in proteinfreiem Medium, dadurch gekennzeichnet, daß man Kulturoberflächen aus Polyvinylformal und/oder Polyvinylbutyral verwendet.

2. Verfahren nach Anspruch 1, dadurch gekennzeichnet, daß man Polyvinylformal als Kulturoberfläche verwendet.

3. Verfahren nach Anspruch 1 oder 2, dadurch gekennzeichnet, daß die Zellen als kontinuierliche Monolayer kultiviert werden.

4. Verfahren nach Anspruch 1 bis 3, dadurch gekennzeichnet, daß die Zellen mit Viren infiziert werden oder auf natürliche oder rekombinierte Weise biologische Substanzen produzieren.

5. Verfahren nach Anspruch 4, dadurch gekennzeichnet, daß die Zellen ursprünglich bei einer Zelldichte von mindestens 2.0x10⁴ Zellen/cm² auf die Oberfläche geimpft werden.

6. Die Verwendung eines Bioreaktors zur Kultivierung von Vero-Zellen in proteinfreiem Medium, dadurch gekennzeichnet, daß der Bioreaktor eine Kulturoberfläche aus Polyvinylformal und/oder Polyvinylbutyral enthält.

7. Die Verwendung eines Bioreaktors nach Anspruch 6, dadurch gekennzeichnet, daß die Kulturoberfläche aus Polyvinylformal und/oder Polyvinylbutyral aus einer Wand des Reaktors und/oder aus der Oberfläche von Füllungskörpern besteht.

## Claims

1. Process for cultivating vero cells in a protein-free medium, characterised in that culture surfaces consisting of polyvinylformal and/or polyvinylbutyral are used.

2. Process according to claim 1, characterised in that polyvinylformal is used as the culture surface.

3. Process according to claim 1 or 2, characterised in that the cells are cultivated as a continuous monolayer.

4. Process according to claims 1 to 3, characterised in that the cells are infected with viruses or produce biological substances by a natural or recombinant method.

5. Process according to claim 4, characterised in that the cells are originally seeded onto the surface in a cell density of at least 2.0 x10⁴ cells/cm².

6. The use of a bioreactor for cultivating vero cells in protein-free medium, characterised in that the bioreactor contains a culture surface of polyvinylformal and/or polyvinylbutyral.

7. The use of a bioreactor according to claim 6, characterised in that the culture surface of polyvinylformal and/or polyvinylbutyral consists of a wall of the reactor and/or the surface of filling means.

## Revendications

1. Procédé de culture de cellules Vero dans un milieu sans protéines, caractérisé en ce que l'on utilise des surfaces de culture en poly(formal vinylique) et/ou en poly(butyral vinylique).

2. Procédé selon la revendication 1, caractérisé en ce que l'on utilise du poly(formal vinylique) comme surface de culture.

3. Procédé selon la revendication 1 ou 2, caractérisé en ce que les cellules sont cultivées sous forme de monocouche continue.

4. Procédé selon les revendications 1 à 3, caractérisé en ce que les cellules sont infectées par des virus ou produisent des substances biologiques de manière naturelle ou par recombinaison.

5. Utilisation selon la revendication 4, caractérisée en ce que les cellules sont inoculées initialement sur la surface à une densité cellulaire d'au moins 2,0 x 10⁴ cellules/cm².

6. Utilisation d'un bioréacteur pour la culture de cellules Vero dans un milieu sans protéines, caractérisée en ce que le bioréacteur contient une surface de culture en poly(formal vinylique) et/ou en poly(butyral vinylique).

7. Utilisation d'un bioréacteur selon la revendication 6, caractérisée en ce que la surface de culture en poly(formal vinylique) et/ou en poly(butyral vinylique) consiste en une paroi du réacteur et/ou en la surface de corps de remplissage.
